# EUROPEAN PATENT APPLICATION

(11) **EP 4 692 789 A1**
(43) Date of publication of application: **11.02.2026**
(21) Application number: 24817824.6
(22) Date of filing: 27.11.2024
(51) Int. Cl.: G01N 33/00, G01N 1/22

(54) **CO2 PURITY MEASUREMENT SYSTEM**

(30) Priority: 06.05.2024 CN 202410550969; 30.10.2024 CN 202411534734
(71) Applicant: China Institute of Atomic Energy, Beijing 102413 (CN)
(72) Inventor: YANG, Wanhuan, Beijing 102413 (CN); QIAO, Pengrui, Beijing 102413 (CN); ZHAO, Xueli, Beijing 102413 (CN); WANG, Kui, Beijing 102413 (CN); WANG, Jingchun, Beijing 102413 (CN); LI, Shen, Beijing 102413 (CN); ZOU, Jichun, Beijing 102413 (CN); WANG, Zhihao, Beijing 102413 (CN); WANG, Minghao, Beijing 102413 (CN); CHEN, Mengyao, Beijing 102413 (CN); PENG, Dequan, Beijing 102413 (CN); ZHU, Qingfu, Beijing 102413 (CN); YANG, Wen, Beijing 102413 (CN); ZHONG, Weihua, Beijing 102413 (CN)
(74) Representative: Isarpatent
(86) International application number: PCT/CN2024/135032
(87) International publication number: WO 2025/232157

(57) **Abstract**

The embodiments of the present application relate to testing or analyzing a material by means of determining chemical or physical properties of the material, and particularly to a system for measuring a CO₂ purity. The system comprises a sample introduction pipeline for receiving CO₂ to be measured and at least one group of impurity ingredient measuring components. Each group of impurity ingredient measuring components comprise a switching element and two impurity ingredient measuring elements for measuring an impurity content. The two impurity ingredient measuring elements in the same group have different measuring ranges, and the switching element is configured to determine whether to switch the fluid communication with the sample introduction pipeline to the other impurity ingredient measuring element in the same group according to the measuring range of the impurity ingredient measuring element currently being in fluid communication with the sample introduction pipeline and an impurity content measured thereby. The system provided by the embodiments of the present application measures the impurity content by switching between the impurity ingredient measuring elements having different measuring ranges via the switching element to measure the impurity content, which is advantageous for increasing the accuracy and precision of the measurement result.

## Description

### Technical Field

The embodiments of the present application relate to the field of testing or analyzing a material by means of determining chemical or physical properties of the material, and particularly to a system for measuring a CO₂ purity.

### Background

The description here merely provides background information relating to the present application, but does not necessarily form prior art.

Common methods for measuring a CO₂ purity comprise an absorption method, an inversion measurement method, and a gas chromatography method. Here, in the absorption method, CO₂ is absorbed by an alkaline solution by using the weak acidity of CO₂, and the CO₂ purity is determined through the volume difference before and after the absorption. In the inversion measurement method, the CO₂ purity is determined by subtracting the impurity content in the CO₂ from 100%. In the gas chromatography method, CO₂ is separated by using difference in separating conditions between CO₂ and other components and detected, so as to determine the CO₂ purity.

### Summary

A brief summary on the present application is presented below to provide a basic understanding of certain aspects of the present application. It should be understood that this summary is not intended to exclusively describe the present application. It is neither intended to determine the critical or essential parts of the present application, nor intended to limit the scope of the present application. The purpose is merely to provide some concepts in a simplified way, which serve as a preamble part for further detailed description discussed later.

The embodiments of the present application provide A system for measuring a CO₂ purity, wherein the system comprises: a sample introduction pipeline for receiving a CO₂ sample gas to be measured; and at least one group of impurity ingredient measuring components, each group of impurity ingredient measuring components comprising a switching element and two impurity ingredient measuring elements for measuring a content of one and the same kind of impurity in the CO₂ sample gas in the sample introduction pipeline, wherein the two impurity ingredient measuring elements in the same group have different measuring ranges, the switching element is used to select one of the two impurity ingredient measuring elements in the same group to be in fluid communication with the sample introduction pipeline, the switching element is configured to determine whether to switch the fluid communication with the sample introduction pipeline to the other impurity ingredient measuring element in the same group according to the measuring range of the impurity ingredient measuring element currently being in fluid communication with the sample introduction pipeline and a corresponding impurity content in the CO₂ sample gas measured thereby; wherein the switching element is a three-way valve comprising one inlet end and two outlet ends, wherein the inlet end is in fluid communication with the sample introduction pipeline, and the two outlet ends are in fluid communication with the two impurity ingredient measuring elements in the same group respectively; the system further comprises: a pressure measuring element for measuring a pressure value of the CO₂ sample gas entering the sample introduction pipeline; and a pressure adjusting element for adjusting a pressure of the CO₂ sample gas entering the sample introduction pipeline according to the pressure value measured by the pressure measuring element such that the pressure of the CO₂ sample gas is within a predetermined range; the system further comprises: a controller for controlling the switching element in each group of impurity ingredient measuring components to switch the fluid communication with the sample introduction pipeline to an impurity ingredient measuring element having a corresponding measuring range according to a type of the CO₂ sample gas to be measured; wherein the controller is preset with types of the CO₂ sample gas as well as impurity ingredients and content ranges of the impurity ingredients corresponding to various types; the controller may further comprise an input module for inputting a type of the CO₂ sample gas, and the controller matches the input type of the CO₂ sample gas against the impurity ingredients and the content ranges of the impurity ingredients corresponding to the various types preset therein, so as to control the switching element to switch fluid communication with the sample introduction pipeline to the impurity ingredient measuring element having a corresponding measuring range in each group of impurity ingredient measuring components.

The system provided by the embodiments of the present application measures the impurity content by switching between the impurity ingredient measuring elements having different measuring ranges via the switching element to measure the impurity content, which is advantageous for increasing the accuracy and precision of the measurement result.

### Brief Description of Drawings

Fig. 1 is a schematic structure diagram of a system for measuring a CO₂ purity provided by the embodiments of the present application.
Fig. 2 is a partially enlarged view of the region A in Fig. 1.
Fig. 3 is a schematic diagram showing the principle of a supercritical CO₂ corrosion test device according to an embodiment of the present application.
Fig. 4 is a schematic sectional view of a sample loading device according to an embodiment of the present application.
Fig. 5 is a schematic partially enlarged view of the sample chamber of the sample loading device in Fig. 4.
Fig. 6 is a schematic sectional view of a sample holding component according to an embodiment of the present application.
Fig. 7 is a schematic exploded view of the sample holding component according to an embodiment of the present application.
Fig. 8 is a schematic top view of a holding element according to an embodiment of the present application.

### Reference number list:

100: measuring system;
10: impurity ingredient measuring component; 11: CO measuring component; 111: first switching element; 1111: inlet end; 1112: outlet end; 112: high range CO measuring element; 113: low range CO measuring element; 114: CO measuring component exhaust port; 12: O₂ measuring component; 121: second switching element; 1211: inlet end; 1212: outlet end; 122: O₂ measuring element having a high measuring range; 123: O₂ measuring element having a low measuring range; 124: O₂ measuring component exhaust port; 13: CₙHₘ measuring component; 131: third switching element; 1311: inlet end; 1312: outlet end; 132: high range CₙHₘ measuring element; 133: low range CₙHₘ measuring element; 134: CₙHₘ measuring component exhaust port; 101: first flow rate measuring element; 102: second flow rate measuring element; 103: third flow rate measuring element;
20: pressure measuring element;
31: pressure reducing valve; 32: safety valve; 33: metal adjusting valve; 40: water content measuring element; 41: water content measuring element exhaust port; 50: drying element; 60: filter element; 70: temperature measuring element; 80: controller;
91: sample introduction pipeline; 911: first valve; 912: measured sample gas inlet; 92: second pipeline; 921: second valve; 93: third pipeline; 931: fourth switch valve; 9311: inlet end; 9312: outlet end; 94: fourth pipeline; 95: fifth pipeline; 96: standard sample gas introduction pipeline; 961: sixth valve; 962: standard sample gas inlet; 97: purge pipeline; 971: seventh valve; 972: fourth flow rate measuring element; 973: purge gas inlet; 98: eighth pipeline; 99: pressure adjusting pipeline; 991: exhaust port;
21: sample loading device; 210: sample chamber; 220: gas introduction opening; 230: gas discharge opening; 240: sample holding component; 241: holding element; 2411: positioning groove group; 24111: positioning groove; 241111: center groove area; 241112: end groove area; 2412: core portion; 2413: extending portion; 2414: center mounting hole; 2415: holding element outside mounting hole; 242: connecting assembly; 2421: connecting nesting element; 24211: nesting element body; 24212: clamping portion; 2422: connection fitting element; 2423: fastening element; 2424: gasket; 24241: gasket mounting hole; 2425: connection element; 250: sample; 260: rotation driving element; 261: rotation shaft; 2611: rotation shaft connecting port; 262: rotation cylinder; 2621: magnet; 2622: connection cylinder; 263: rotation cylinder cooling element; 264: rotation shaft cooling element; 2641: first cooling connection portion; 2642: second cooling connection portion; 2643: cooling body; 265: drive mounting element; 266: sleeve; 2661: first connection portion; 2662: second connection portion; 267: drive element; 268: bearing; 270: body; 2701: groove; 271: cover; 2711: through hole; 272: fixing element; 273: static sample groove; 274: fastening element; 275: bearing bracket; 280: pressure adjusting valve; 281: first temperature measuring element; 282: first electric heating element; 290: auxiliary pressure adjusting element; 291: auxiliary pressure adjusting valve; 292: pressure stabilization vessel; 293: pressure measuring element; 294: auxiliary temperature measuring element; 295: auxiliary electric heating element; 2101: carbon dioxide gas supply; 2102: gas source valve; 2103: displacement valve; 2104: first pressure measuring element; 2105: precooling element; 2106: cool water supply element; 2107: filter element; 2110: gas introduction pipeline; 2121: booster pump; 2122: valve; 2123: second pressure measuring element; 2124: check valve; 2131: preheating element; 2132: temperature control element; 2141: cooling element; 2142: second temperature measuring element; 2143: third pressure measuring element; 2144: vent valve; 2145: rupture disc; 2146: atmospheric relief valve.

### Detailed Description

The exemplary embodiments of the present application will be described below with reference to the drawings. For the purpose of clarity and simplicity, not all the features of the practical embodiments will be described in the specification. However, it should be understood that during the development of any of such practical embodiments, many determinations specific to the embodiments should be made to achieve the particular goal of the developer, for example, to meet those limiting conditions related to the system and business, and those limiting conditions may vary depending on various embodiments. Furthermore, it should also be understood that although the development work may be very complicated and time-consuming, such development work is merely a routine task for those skilled in the art benefiting from the disclosure of the present application.

Furthermore, it should be noted that in order to avoid obscuring the present application due to unnecessary details, only the device structures and/or process steps closely associated to the embodiments of the present application are shown in the drawings, while other details which are not relevant to the present application are omitted.

It should be noted that unless otherwise defined, the technical terms or scientific terms used in the present application should have usual meanings understood by a person of ordinary skills in the art.

In the description of the embodiments of the present application, unless specifically and particularly defined otherwise, "a plurality of" refers to at least two, for example, two, three and so on.

The inversion measurement method is a common method for measuring the purity of highly pure CO₂. When measuring the CO₂ purity according to the inversion measurement method, the system for measuring a CO₂ purity in related technologies is only provided with one measuring element. When the content of the impurity ingredient in CO₂ is low (for example, the content of the impurity ingredient in CO₂ is much less than the maximum measuring range of the measuring element), inaccurate measurement result is likely to occur. When the content of the impurity ingredient in CO₂ is high, it is likely to occur that the content of the impurity ingredient exceeds the measuring range of the measuring element.

In view of the above, the embodiments of the present application provide a system for measuring a CO₂ purity (hereinafter simply referred to as a measuring system).

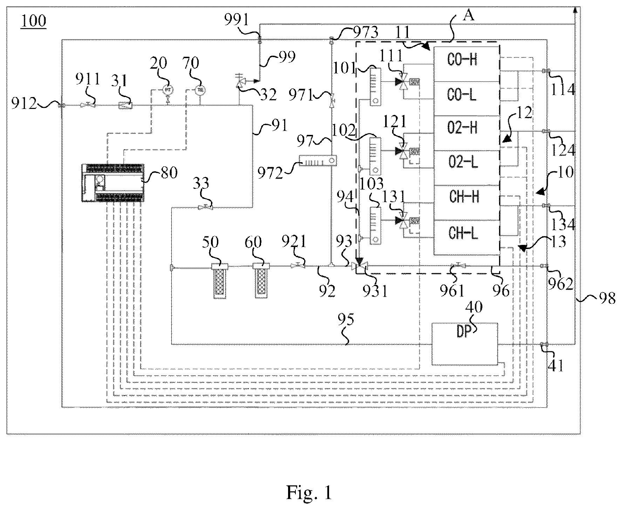

Fig. 1 shows a schematic structure diagram of the measuring system provided by the embodiments of the present application. As shown in Fig. 1, the measuring system 100 comprises a sample introduction pipeline 91 and at least one group of impurity ingredient measuring components 10. The sample introduction pipeline 91 is used for receiving a CO₂ sample gas to be measured. Each group of impurity ingredient measuring components 10 comprise a switching element and two impurity ingredient measuring elements for measuring a content of one and the same kind of impurity in the CO₂ sample gas in the sample introduction pipeline 91. Here, the two impurity ingredient measuring elements in the same group have different measuring ranges, the switching element is used to select one of the two impurity ingredient measuring elements in the same group to be in fluid communication with the sample introduction pipeline 91, the switching element is configured to determine whether to switch the fluid communication with the sample introduction pipeline 91 to the other impurity ingredient measuring element in the same group according to the measuring range of the impurity ingredient measuring element currently being in fluid communication with the sample introduction pipeline 91 and a corresponding impurity content in the CO₂ sample gas measured thereby.

The measuring system 100 provided by the embodiments of the present application determines the CO₂ purity by measuring the content of the impurity; continuous measurement on the CO₂ purity can be performed by switching between the impurity ingredient measuring elements having different measuring ranges via the switching element, which is advantageous for increasing the accuracy and precision of the measurement result.

In some embodiments, the CO₂ purity of the CO₂ sample gas to be measured may be in a range of 99.9-99.99%.

In some embodiments, the switching element may be a three-way valve comprising one inlet end and two outlet ends, wherein the inlet end is in fluid communication with the sample introduction pipeline 91, and the two outlet ends are in fluid communication with the two impurity ingredient measuring elements in the same group respectively. When measuring the corresponding impurity content in the CO₂ sample gas, the inlet end is in fluid communication with one of the two outlet ends, and it may be determined whether to switch the inlet end to be in fluid communication with the other outlet end according to the measuring range of the impurity ingredient measuring element currently being in fluid communication with the sample introduction pipeline 91 and a corresponding impurity content in the CO₂ sample gas measured thereby.

Referring to Fig. 1, in some embodiments, the measuring system 100 may further comprise a pressure measuring element 20 and a pressure adjusting element. Here, the pressure measuring element 20 is used for measuring a pressure value of the CO₂ sample gas entering the sample introduction pipeline 91; and the pressure adjusting element is used for adjusting a pressure of the CO₂ sample gas entering the sample introduction pipeline 91 according to the pressure value measured by the pressure measuring element 20 such that the pressure of the CO₂ sample gas is within a predetermined range. In some embodiments, the pressure measuring element 20 may be a pressure meter.

In some embodiments, the pressure adjusting element may comprise a pressure reducing valve 31 and a metal adjusting valve 33, wherein the pressure reducing valve 31 and the metal adjusting valve 33 are disposed in the sample introduction pipeline 91, and the metal adjusting valve 33 is disposed downstream of the pressure reducing valve 31. The pressure reducing valve 31 can greatly reduce the pressure of the CO₂ sample gas entering the sample introduction pipeline 91, and the metal adjusting valve 33 can finely tune the pressure of the CO₂ sample gas entering the sample introduction pipeline 91.

In some embodiments, the measuring system 100 may comprise a pressure adjusting pipeline 99, wherein one end of the pressure adjusting pipeline 99 is in communication with the sample introduction pipeline 91, and the other end is in fluid communication with the eighth pipeline 98 via an exhaust port 991, and a gas flowing into the pressure adjusting pipeline 99 may be discharged to the atmospheric environment via the exhaust port 991 and the eighth pipeline 98. The pressure adjusting element may further comprise a safety valve 32 disposed in the pressure of the pipeline 99. When the pressure of the CO₂ sample gas in the sample introduction pipeline 91 which has been reduced by the pressure reducing valve 31 exceeds the threshold, the safety valve 32 will be opened to open the pressure adjusting pipeline 99. At this time, the gas in the sample introduction pipeline 91 is discharged to the atmospheric environment via the pressure adjusting pipeline 99 so as to ensure that the pressure of the gas flowing out of the sample introduction pipeline 91 is within an appropriate range.

In some embodiments, the pressure adjusting pipeline 99 may be in fluid communication with the sample introduction pipeline 91 between the downstream of the pressure reducing valve 31 and the upstream of the metal adjusting valve 33.

A portion of the gas in the sample introduction pipeline 91 can be discharged through the pressure adjusting pipeline 99 and the safety valve 32, which is advantageous for adjusting the pressure of the CO₂ sample gas entering the sample introduction pipeline 91, such that the pressure of the CO₂ sample gas is within a predetermined range.

In related technologies, the source of the CO₂ sample gas connected to the measured sample gas inlet 912 of the sample introduction pipeline 91 has a high pressure, such that the pressure at the measured sample gas inlet 912 is excessively high, and a relatively high gas pressure will influence the impurity ingredient measuring elements. With respect to the problem of excessively high pressure at the measured sample gas inlet 912, the inventors of the present application have carried out multiple tests and measurements and found that the pressure of the CO₂ sample gas can be adjusted to be within a predetermined range through multiple adjustments and control by adding the pressure reducing valve 31 and the metal adjusting valve 33 in the sample introduction pipeline 91, providing the pressure adjusting pipeline 99, and providing the safety valve 32 in the pressure adjusting pipeline 99, and the pressure at the sample gas inlet can be ensured to be maintained within a predetermined range by monitoring in a remote system achieved by the controller 80 provided hereinafter by providing the pressure measuring element 20.

Referring to Fig. 1, in some embodiments, the measuring system 100 may comprise a plurality of groups of impurity ingredient measuring components 10 for measuring different kinds of impurity ingredients in a CO₂ sample gas respectively, wherein the impurity ingredients include at least one of CO, O₂ and CₙHₘ, and the plurality of groups of impurity ingredient measuring components 10 are disposed in parallel with each other so as to simultaneously measure different kinds of impurity ingredients in the CO₂ sample gas, which is advantageous for ensuring the accuracy of the measurement result.

CO, O₂ and CₙHₘ are main impurity ingredients in CO₂, and the CO₂ purity may be obtained by measuring the content of the impurity ingredient and subtracting the content of the impurity from 100%. The plurality of groups of impurity ingredient measuring components 10 are disposed in parallel with each other, such that they may measure the contents of CO, O₂ and CₙHₘ respectively, which is advantageous for preventing the impurity ingredients from influencing each other, and increasing the accuracy of the impurity ingredient measurement.

In some embodiments, the measuring system 100 may further comprise a plurality of measuring branches, wherein those measuring branches are in fluid communication with the fourth pipeline 94 in parallel, and each of the impurity ingredient measuring components 10 is disposed in one corresponding measuring branch.

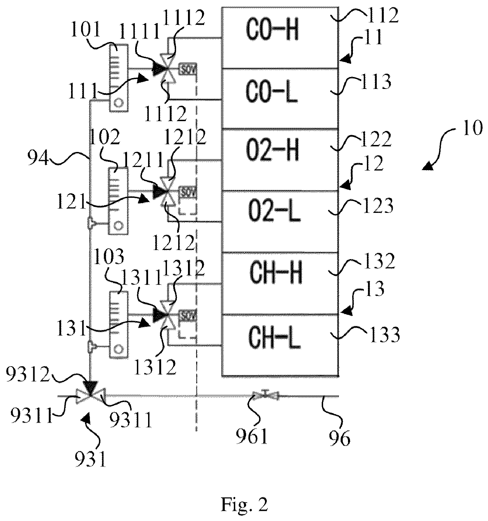

Fig. 2 shows a partially enlarged view of the region A in Fig. 1. As shown in Fig. 2, in some embodiments, the impurity ingredient measuring components 10 may comprise a CO measuring component 11 for measuring a CO content in a CO₂ sample gas. The CO measuring component 11 may be disposed in the first measuring branch.

Referring to Fig. 1, in some embodiments, the CO measuring component 11 may comprise a first switching element 111, a high range CO measuring element 112 and a low range CO measuring element 113, wherein the first switching element 111 is used to select one of the high range CO measuring element 112 and the low range CO measuring element 113 to be in fluid communication with the sample introduction pipeline 91. For example, when the CO content in the CO₂ sample gas falls within the measuring range of the low range CO measuring element 113, the low range CO measuring element 113 is selected for measurement, and the low range CO measuring element 113 is in fluid communication with the sample introduction pipeline 91 through the first switching element 111; and when the CO content in the CO₂ sample gas exceeds the measuring range of the low range CO measuring element 113, the high range CO measuring element 112 is selected for measurement, and the high range CO measuring element 112 is in fluid communication with the sample introduction pipeline 91 through the first switching element 111.

In some embodiments, the high range CO measuring element 112 may be a CO high range analyzer with a measuring range of 100 to 2000 ppm; the low range CO measuring element 113 may be a CO low range analyzer with a measuring range of 0 to 100 ppm; and the first switching element 111 may be an electromagnetic three-way switching valve.

Referring to Fig. 2, in some embodiments, the impurity ingredient measuring components 10 may comprise an O₂ measuring component 12 for measuring an O₂ content in a CO₂ sample gas. The O₂ measuring component 12 may be disposed in the second measuring branch.

Referring to Fig. 1, in some embodiments, the O₂ measuring component 12 may comprise a second switching element 121, a high range O₂ measuring element 122 and a low range O₂ measuring element 123, wherein the second switching element 121 is used to select one of the high range O₂ measuring element 122 and the low range O₂ measuring element 123 to be in fluid communication with the sample introduction pipeline 91. For example, when the O₂ content in the CO₂ sample gas falls within the measuring range of the low range O₂ measuring element 123, the low range O₂ measuring element 123 is selected for measurement, and the low range O₂ measuring element 123 is in fluid communication with the sample introduction pipeline 91 through the second switching element 121; and when the O₂ content in the CO₂ sample gas exceeds the measuring range of the low range O₂ measuring element 123, the high range O₂ measuring element 122 is selected for measurement, and the high range O₂ measuring element 122 is in fluid communication with the sample introduction pipeline 91 through the second switching element 121.

In some embodiments, the high range O₂ measuring element 122 may be an O₂ analyzer with a measuring range of 0-5%; and the low range O₂ measuring element may be an O₂ microanalyzer with a measuring range of 0-100-1000 ppm, wherein the O₂ microanalyzer may automatically switch between the large and small measuring ranges; and the second switching element 121 may be an electromagnetic three-way switching valve.

Referring to Fig. 2, in some embodiments, the plurality of groups of impurity ingredient measuring components 10 may comprise a CₙHₘ measuring component 13 for measuring a CₙHₘ content in the CO₂ sample gas. The CₙHₘ measuring component 13 may be disposed in the third measuring branch.

Referring to Fig. 1, in some embodiments, the CₙHₘ measuring component 13 may comprise a third switching element 131, a high range CₙHₘ measuring element 132 and a low range CₙHₘ measuring element 133, wherein the third switching element 131 is used to select one of the high range CₙHₘ measuring element 132 and the low range CₙHₘ measuring element 133 to be in fluid communication with the sample introduction pipeline 91. For example, when the CₙHₘ content in the CO₂ sample gas falls within the measuring range of the low range CₙHₘ measuring element 133, the low range CₙHₘ measuring element 133 is selected for measurement, and the low range CₙHₘ measuring element 133 is in fluid communication with the sample introduction pipeline 91 through the third switching element 131; and when the CₙHₘ content in the CO₂ sample gas exceeds the measuring range of the low range CₙHₘ measuring element 133, the high range CₙHₘ measuring element 132 is selected for measurement, and the high range CₙHₘ measuring element 132 is in fluid communication with the sample introduction pipeline 91 through the third switching element 131.

In some embodiments, the high range CₙHₘ measuring element 132 may be a CₙHₘ high range analyzer with a measuring range of 100 to 2000 ppm; the low range CₙHₘ measuring element 133 may be a CₙHₘ low range analyzer with a measuring range of 0 to 100 ppm; and the third switching element 131 may be an electromagnetic three-way switching valve.

In some embodiments, the measuring system 100 may further comprise a water content measuring element 40 for measuring a water content in a CO₂ sample gas. In some embodiments, the water content measuring element 40 may be a dew point analyzer (DP analyzer). The dew point analyzer is operated at a temperature in a range from -100°C to +20°C.

Referring to Fig. 1, in some embodiments, the sample introduction pipeline 91 is provided with a first valve 911 and a measured sample gas inlet 912. The CO₂ sample gas to be measured may enter into the sample introduction pipeline 91 through the measured sample gas inlet 912.

Referring to Fig. 1, in some embodiments, the measuring system 100 may further comprise a second pipeline 92, a fourth pipeline 94 and a fifth pipeline 95. The second pipeline 92 and the fifth pipeline 95 are in fluid communication with the sample introduction pipeline 91 in parallel. The impurity ingredient measuring components 10 are in parallel with each other and in fluid communication with the fourth pipeline 94, and the fourth pipeline 94 is in fluid communication with the second pipeline 92. The fifth pipeline 95 is in fluid communication with the water content measuring element 40. The second pipeline 92 is provided with a second valve 921, and the third pipeline 93 is provided with a fourth switching valve 931.

After the CO₂ sample gas enters the sample introduction pipeline 91, a portion thereof enters each of the impurity ingredient measuring components 10 via the second pipeline 92 and the fourth pipeline 94; and the other portion thereof enters the water content measuring element 40 via the fifth pipeline 95.

Referring to Fig. 1, in some embodiments, the measuring system 100 may further comprise a drying element 50, wherein the drying element 50 is used for drying the CO₂ sample gas in the sample introduction pipeline 91 such that the dried CO₂ sample gas enters the at least one group of impurity ingredient measuring components 10. In some embodiments, the drying element 50 may be a dryer provided with a removable filter cartridge for drying. The drying element 50 can remove the moisture in the CO₂ sample gas, so as to prevent the moisture in the CO₂ sample gas from influencing the accuracy of the measurement results of other impurity ingredients.

In some embodiments, the measuring system 100 may further comprise a filter element 60, wherein the filter element 60 is used for filtering the CO₂ sample gas in the sample introduction pipeline 91 such that the filtered CO₂ sample gas enters the at least one group of impurity ingredient measuring components 10. In some embodiments, the filter element 60 may be a filter, and the filter element 60 may remove the solid impurity in the CO₂ sample gas by filtration, so as to prevent the solid impurity from influencing the accuracy of the measurement results of the impurities.

In some embodiments, the drying element 50 and the filter element 60 may be disposed in the second pipeline 92, such that they will not influence the detection of the moisture in the CO₂ sample gas by the water content measuring element 40.

In some embodiments, the pressure measuring element 20, the pressure reducing valve 31 and the metal adjusting valve 33 may be disposed in the sample introduction pipeline 91.

Referring to Fig. 2, in some embodiments, the inlet end 1111 of the first switching element 111, the inlet end 1211 of the second switching element 121 and the inlet end 1311 of the third switching element 131 are in fluid communication with three measuring branches respectively, two outlet ends 1112 of the first switching element 111 are in fluid communication with two CO measuring elements respectively, two outlet ends 1212 of the second switching element 121 are in fluid communication with two O₂ measuring elements respectively, and two outlet ends 1312 of the third switching element 131 are in fluid communication with two CₙHₘ measuring elements respectively.

In some embodiments, the measuring system 100 may further comprise a first flow rate measuring element 101. The first flow rate measuring element 101 may be disposed in the first measuring branch to measure the flow rate of a gas flowing into the CO measuring component 11. The first flow rate measuring element 101 may be disposed upstream of the first switching element 111.

In some embodiments, the measuring system 100 may further comprise a second flow rate measuring element 102. The second flow rate measuring element 102 may be disposed in the second measuring branch to measure the flow rate of a gas flowing into the O₂ measuring component 12. The second flow rate measuring element 102 may be disposed upstream of the second switching element 121.

In some embodiments, the measuring system 100 may further comprise a third flow rate measuring element 103. The third flow rate measuring element 103 may be disposed in the third measuring branch to measure the flow rate of a gas flowing into the CₙHₘ measuring component 13. The third flow rate measuring element 103 may be disposed upstream of the third switching element 131.

In some embodiments, the first flow rate measuring element 101, the second flow rate measuring element 102 and the third flow rate measuring element 103 may all be a flowmeter.

Referring to Fig. 1, in some embodiments, the measuring system 100 may further comprise a standard sample gas introduction pipeline 96 for providing a standard sample having a known impurity content to the at least one group of impurity ingredient measuring components 10 so as to calibrate the at least one group of impurity ingredient measuring components 10.

In some embodiments, the standard sample gas introduction pipeline 96 is provided with a sixth valve 961 and a standard sample gas inlet 962, wherein the standard sample gas inlet 962 is disposed at one end of the standard sample gas introduction pipeline 96, and the other end of the standard sample gas introduction pipeline 96 is in fluid communication with the fourth pipeline 94 through the fourth switching valve 931; and the standard sample entered from the standard sample gas inlet 962 can flow through the standard sample gas introduction pipeline 96 and the fourth pipeline 94 to the impurity ingredient measuring components 10. Referring to Fig. 1, in some embodiments, the measuring system 100 may further comprise a purge pipeline 97 for providing a purge gas to the sample introduction pipeline 91 and the at least one group of impurity ingredient measuring components 10. The purge gas may protect the meters and devices in the measuring system 100, thereby improving the service life of the meters and devices. In some embodiments, the purge gas may be nitrogen. In some embodiments, when no CO₂ purity measurement is performed, the purge gas may be provided to the sample introduction pipeline 91 and the impurity ingredient measuring components 10 for purging.

In some embodiments, the purge pipeline 97 is provided with a seventh valve 971, a fourth flow rate measuring element 972 and a purge gas inlet 973, wherein the purge gas inlet 973 is disposed at one end of the purge pipeline 97, the other end of the purge pipeline 97 is in fluid communication with the fourth pipeline 94 and the second pipeline 92 through the third pipeline 93 and the fourth switching valve 931; the purge gas entered from the purge gas inlet 973 can flow through the purge pipeline 97 into the third pipeline 93, the fourth pipeline 94 and each measuring branches, and the purge gas can also flow through the purge pipeline 97 into the second pipeline 92 and the sample introduction pipeline 91.

In some embodiments, the fourth switching valve 931 may be an electromagnetic three-way switching valve, and the fourth switching valve 931 comprises two inlet ends 9311 and one outlet end 9312, wherein the two inlet ends 9311 are in fluid communication with the third pipeline 93 and the purge pipeline 97 respectively, and the outlet end 9312 is in fluid communication with the fourth pipeline 94.

In some embodiments, the fourth flow rate measuring element 972 may be a flowmeter, and the fourth flow rate measuring element 972 may be used for measuring the flow rate of the purge gas entered through the purge gas inlet 973.

Referring to Fig. 1, in some embodiments, the measuring system 100 may further comprise a discharge pipeline for discharging the gas from the at least one group of impurity ingredient measuring components 10 to the atmospheric environment. Because the main ingredient of the gas from the impurity ingredient measuring components 10 is CO₂, the gas from the impurity ingredient measuring components 10 is discharged to the atmospheric environment through the discharge pipeline, which can avoid the influence of excessive high concentration of CO₂ on the safety of an operator.

In some embodiments, the impurity ingredient measuring components 10 may further comprise a CO measuring component exhaust port 114, an O₂ measuring component exhaust port 124 and a CₙHₘ measuring component exhaust port 134. The CO measuring component exhaust port 114 is in fluid communication with the two CO measuring elements, and is used for discharging gas from the two CO measuring elements. The O₂ measuring component exhaust port 124 is in fluid communication with the two O₂ measuring elements, and is used for discharging gas from the two O₂ measuring elements. The CₙHₘ measuring component exhaust port 134 is in fluid communication with the two CₙHₘ measuring elements, and is used for discharges gas from the two CₙHₘ measuring elements.

In some embodiments, the measuring system 100 may further comprise an eighth pipeline 98, wherein the eighth pipeline 98 is in fluid communication with the CO measuring component exhaust port 114, the O₂ measuring component exhaust port 124 and the CₙHₘ measuring component exhaust port 134 and extends to outdoors, and gases discharged from the CO measuring components 11, the O₂ measuring components 12 and the CₙHₘ measuring components 13 may flow into the eighth pipeline 98 via the CO measuring component exhaust port 114, the O₂ measuring component exhaust port 124, and the CₙHₘ measuring component exhaust port 134 respectively, and flow to the outdoor atmospheric environment via the eighth pipeline 98. The eighth pipeline 98 forms a discharge pipeline for discharging gases from various groups of impurity ingredient measuring components 10 to the atmospheric environment.

Referring to Fig. 1, in some embodiments, the water content measuring element 40 may further comprise a water content measuring element exhaust port 41, wherein the water content measuring element exhaust port 41 is in fluid communication with the eighth pipeline 98, and a gas flowing through the water content measuring element 40 may flow through the eighth pipeline 98 via the water content measuring element exhaust port 41, and flow to the outdoor atmospheric environment.

Referring to Fig. 1, in some embodiments, the measuring system 100 may further comprise a temperature measuring element 70, wherein the temperature measuring element 70 may be disposed in the sample introduction pipeline 91, and positioned downstream of the pressure measuring element 20, and the temperature measuring element 70 may be used to measure a temperature value of the CO₂ sample gas entering the sample introduction pipeline 91. In some embodiments, the temperature measuring element 70 may be a temperature measuring instrument.

The pressure measuring element 20 and the temperature measuring element 70 may be disposed downstream of the pressure reducing valve 31, and the pressure adjusting pipeline 99 is in fluid communication with the sample introduction pipeline 91 downstream of the temperature measuring element 70.

Referring to Fig. 1, in some embodiments, the measuring system 100 may further comprise a controller 80, wherein the controller 80 may be used for receiving the CO content, the O₂ content and the CₙHₘ content measured by the impurity ingredient measuring components 10, and the water content measured by the water content measuring element 40. The controller 80 may process and analyze the data received to finally obtain the CO₂ purity.

In some embodiments, the controller 80 may also control the switching element in the corresponding impurity measuring component according to the measuring range of the impurity ingredient measuring elements currently being in fluid communication with the sample introduction pipeline 91 and the corresponding impurity content in the CO₂ sample gas measured thereby. For example, the controller 80 may control whether the first switching element 111 switches the fluid communication with the sample introduction pipeline 91 to the other CO measuring element according to the measuring range of the CO measuring element currently being in fluid communication with the sample introduction pipeline 91 and the corresponding CO content in the CO₂ sample gas.

In some embodiments, the controller 80 may also be used for receiving the pressure value measured by the pressure measuring element 20 and the temperature value measured by the temperature measuring element 70, so as to monitor the measuring system 100 remotely.

In some embodiments, the controller 80 may also control the switching element to switch between the impurity ingredient measuring elements having different measuring ranges. In some embodiments, the controller 80 is preset with types of the CO₂ sample gas and as well as impurity ingredients and content ranges of the impurity ingredients corresponding to the various types. The controller 80 may further comprise an input module for inputting a type of the CO₂ sample gas, and the controller 80 matches the input type of the CO₂ sample gas against the impurity ingredients and the content ranges of the impurity ingredients corresponding to the various types preset therein, so as to control the switching element to switch the fluid communication with the sample introduction pipeline 91 to the impurity ingredient measuring element having a corresponding measuring range in each group of impurity ingredient measuring components 10. As such, in the measurement, by inputting the type of the CO₂ sample gas, the controller 80 can automatically select the impurity ingredient measuring element having a corresponding measuring range. This is advantageous for directly using the impurity ingredient measuring element having a more suitable measuring range to measure a preset kind of impurity, thereby reducing the measurement time.

The embodiments of the present application also provide a method for measuring a CO₂ purity, wherein the method comprises: S1: select an impurity ingredient measuring element having a corresponding measuring range to measure a preset kind of impurity according to a type of a CO₂ sample gas to be measured; and S2: determining whether to switch to another impurity ingredient measuring element having a different measuring range to measure the preset kind of impurity according to a measurement result of the impurity ingredient measuring element currently measuring the CO₂ sample gas.

By selecting the impurity ingredient measuring element having a corresponding measuring range to measure the preset kind of impurity according to the type of the CO₂ sample gas, the method provided by the embodiments of the present application is advantageous for directly using the impurity ingredient measuring element having a more suitable measuring range to measure the preset kind of impurity.

In some embodiments, Step S2 comprises: if the measurement result of the impurity ingredient measuring element currently measuring the CO₂ sample gas shows exceeding the measuring range or shows the current measuring range is large, switching to another impurity ingredient measuring element having a different measuring range to measure the preset kind of impurity.

In some embodiments, the controller 80 is configured to utilize the purge pipeline 97 to purge the sample introduction pipeline 91, the high range CO measuring element 112, the high range O₂ measuring element 122, and the high range CₙHₘ measuring element 132 before purging the low range CO measuring element 113, the low range O₂ measuring element 123, and the low range CₙHₘ measuring element 133. As compared to case where the purge pipeline 97 is utilized to first purge the sample introduction pipeline 91, the low range CO measuring element 113, the low range O₂ measuring element 123, and the low range CₙHₘ measuring element 133, the embodiments of the present application can prevent the influence on the accuracy of the measurement due to the negative effect of the high concentration of impurity ingredients remained in the sample introduction pipeline 91 on various low range measuring elements.

In some embodiments, the method for measuring a CO₂ purity may further comprise: before Step S1, utilizing the purge pipeline 97 to purge the sample introduction pipeline 91, the high range CO measuring element 112, the high range O₂ measuring element 122, and the high range CₙHₘ measuring element 132 before purging the low range CO measuring element 113, the low range O₂ measuring element 123, and the low range CₙHₘ measuring element 133.

Referring to Fig. 1, the process of measuring a CO₂ purity with the measuring system 100 provided by the embodiments of the present application may comprise the following steps (1) to (3).
(1) Before measuring the CO₂ purity in the CO₂ sample gas, a purge gas is firstly utilized to purge the water content measuring element 40 and the impurity ingredient measuring components 10 respectively. First, the water content measuring element 40 id purged. The purge gas enters through the purge gas inlet 973, and the seventh valve 971 and the second valve 921 are opened, and the purge gas flows into the water content measuring element 40 through the purge pipeline 97, the second pipeline 92 and the fifth pipeline 95 to purge the water content measuring element 40, and the gas after purging is discharged into the atmospheric environment via the eighth pipeline 98. During the process, the filter cartridge in the drying element 50 is removed, and after the purging is completed, the filter cartridge reinstalled into the drying element 50. Thereafter, the impurity ingredient measuring components 10 is purged. Because the impurity ingredient measuring components 10 comprises high range measuring elements (i.e., the high range CO measuring element 112, the high range O₂ measuring element 122, and the high range CₙHₘ measuring element 132) and low range measuring elements (i.e., the low range CO measuring element 113, the low range O₂ measuring element 123, and the low range CₙHₘ measuring element 133), the purging of the impurity ingredient measuring components 10 needs to be divided into 2 steps: first purging the high range measuring elements, and then purging the low range measuring elements.
   The high range measuring elements is purged. The first switching element 111, the second switching element 121, and the third switching element 131 are switched to allow the high range CO measuring element 112, the high range O₂ measuring element 122, and the high range CₙHₘ measuring element 132 to be in fluid communication with the sample introduction pipeline 91 respectively. The purge gas enters through the purge gas inlet 973, and the seventh valve 971 is kept open, while the second valve 921 is closed, and the fourth switching valve 931 is switched to allow the third pipeline 93 to be in fluid communication with the fourth pipeline 94, and the purge gas flows from the purge pipeline 97 to the high range measuring elements in the impurity ingredient measuring components 10 through the third pipeline 93 and the fourth pipeline 94, to purge the purge pipeline 97, the third pipeline 93, the fourth pipeline 94 and the high range measuring elements in the impurity ingredient measuring components 10, and the gas after purging is discharged to the atmospheric environment via the eighth pipeline 98. Then the low range measuring elements of the impurity ingredient measuring components 10 are purged. The first switching element 111, the second switching element 121, and the third switching element 131 are switched to allow the low range CO measuring element 113, the low range O₂ measuring element 123, and the low range CₙHₘ measuring element 133 to be in fluid communication with the sample introduction pipeline 91 respectively, and the purging process is the same as that for the high range measuring elements.
(2) After the purging is completed, the CO₂ sample gas to be measured is introduced to begin the measurement. Before the measurement, the type of the CO₂ sample gas is input into the controller 80, the controller 80 matches the input type of the CO₂ sample gas against the impurity ingredients and the content ranges of the impurity ingredients corresponding to the various types preset therein, so as to control each switching element to switch the fluid communication with the sample introduction pipeline 91 to the impurity ingredient measuring element having a corresponding measuring range in each group of impurity ingredient measuring components 10. Thereafter, the CO₂ sample gas to be measured enters through the measured sample gas inlet 912, and the first valve 911 is opened, and the CO₂ sample gas is monitored by the pressure reducing valve 31 according to the pressure measuring element 20 and the temperature measuring element 70 in the sample introduction pipeline 91, and the monitored signal is sent to the controller 80, and the pressure of the CO₂ sample gas to be measured is adjusted to be within a predetermined range by adjusting the pressure reducing valve 31 and the metal adjusting valve 33. After confirming that the installation of the filter cartridges in the drying element 50 and the filter element 60 is completed, the second valve 921 is opened, and the fourth switching valve 931 is switched to allow the third pipeline 93 to be in fluid communication with the fourth pipeline 94. The CO₂ sample gas having a pressure within the predetermined range flows through the second pipeline 92, the third pipeline 93 and the fourth pipeline 94. The first flow rate measuring element 101, the second flow rate measuring element 102, and the third flow rate measuring element 103 are adjusted to be within a predetermined range respectively. The CO₂ sample gas flows through the first flow rate measuring element 101, the second flow rate measuring element 102, and the third flow rate measuring element 103, then through the first switching element 111, the second switching element 121, and the third switching element 131, to the impurity ingredient measuring components 10 to perform the measurement of the impurity ingredients. (The first switching element 111, the second switching element 121, and the third switching element 131 are initially configured to allow the high range CO measuring element 112, the high range O₂ measuring element 122, and the high range CₙHₘ measuring element 132 to be in fluid communication with the sample introduction pipeline 91 respectively. When the measurement starts, the type of the CO₂ sample gas is input into the input module of the controller 80, the controller 80 matches the input type of the CO₂ sample gas against the impurity ingredients and the content ranges of the impurity ingredients corresponding to the various types preset therein, so as to control the first switching element 111, the second switching element 121, and the third switching element 131 to switch the fluid communication with the sample introduction pipeline 91 to the impurity ingredient measuring element having a corresponding measuring range in each group of impurity ingredient measuring components 10 respectively. During the measurement process, the controller 80 may also switch between high and low measuring ranges of various impurity ingredient measuring components 10 according to the content of the CO₂ sample to be measured so as to ensure the precision of the measurement data) Meanwhile, the CO₂ sample gas having a pressure within the predetermined range flows to the water content measuring element 40 through the fifth pipeline 95 to perform measurement of the water contained in the CO₂ sample gas. After the measurements are completed, the gases from the impurity ingredient measuring components 10 and the water content measuring element 40 are discharged to the atmospheric environment via the eighth pipeline 98.
(3) The process of switching the CO measuring components 11 is as follows. When the CO content in the CO₂ sample gas falls within the measuring range of the low range CO measuring element 113, the low range CO measuring element 113 is selected for measurement, and the low range CO measuring element 113 is in fluid communication with the sample introduction pipeline 91 through the first switching element 111. When the CO content in the CO₂ sample gas exceeds the measuring range of the low range CO measuring element 113, the high range CO measuring element 112 is selected for measurement, and the high range CO measuring element 112 is in fluid communication with the sample introduction pipeline 91 through the first switching element 111. The first switching element 111 may be an electromagnetic three-way switching valve which may be controlled by the controller 80 to switch the flow direction of the CO₂ sample gas.
(4) The process of switching the O₂ measuring components 12 is as follows. When the O₂ content in the CO₂ sample gas falls within the measuring range of the low range O₂ measuring element 123, the low range O₂ measuring element 123 is selected for measurement, and the low range O₂ measuring element 123 is in fluid communication with the sample introduction pipeline 91 through the second switching element 121. When the O₂ content in the CO₂ sample gas exceeds the measuring range of the low range O₂ measuring element 123, the high range O₂ measuring element 122 is selected for measurement, and the high range O₂ measuring element 122 is in fluid communication with the sample introduction pipeline 91 through the second switching element 121. The second switching element 121 may be an electromagnetic three-way switching valve which may be controlled by the controller 80 to switch the flow direction of the CO₂ sample gas.
(5) The process of switching the CₙHₘ measuring components 13 is as follows. When the CₙHₘ content in the CO₂ sample gas falls within the measuring range of the low range CₙHₘ measuring element 133, the low range CₙHₘ measuring element 133 is selected for measurement, and the low range CₙHₘ measuring element 133 is in fluid communication with the sample introduction pipeline 91 through the third switching element 131. When the CₙHₘ content in the CO₂ sample gas exceeds the measuring range of the low range CₙHₘ measuring element 133, the high range CₙHₘ measuring element 132 is selected for measurement, and the high range CₙHₘ measuring element 132 is in fluid communication with the sample introduction pipeline 91 through the third switching element 131. The third switching element 131 may be an electromagnetic three-way switching valve which may be controlled by the controller 80 to switch the flow direction of the CO₂ sample gas.
(6) After the measurement is completed, the measurement result of the impurity ingredient and the measurement result of the water content are sent to the controller 80, and the data are processed and analyzed in the controller 80 to obtain the CO₂ purity in the CO₂ sample gas to be measured. The controller 80 has an interface for communication with an external monitor system, which may transmit the measured data to a remote monitoring platform for real time monitoring.

During the process of operating the measuring system 100, when the pressure of the system exceeds the predetermined range for the system, the safety valve 32 will be turned on to release the gas in the pipelines of the system, and the gas will be discharged to the atmospheric environment through the pressure adjusting pipeline 99 to provide an overpressure protection to the system.

In some embodiments, the measuring system 100 may further comprise a supercritical CO₂ corrosion test device, wherein the CO₂ sample gas received by the sample introduction pipeline 91 is from the supercritical CO₂ corrosion test device, and the impurity ingredient measuring components 10 is used to measure the CO₂ purity in the supercritical CO₂ corrosion test device. The controller 80 may adjust the flow rate of the supercritical CO₂ in the supercritical CO₂ corrosion test device according to the CO₂ purity to replace the carbon dioxide in the supercritical CO₂ corrosion test device, so as to adjust the carbon dioxide purity in the supercritical CO₂ corrosion test device, such that the carbon dioxide purity in the supercritical CO₂ corrosion test device is within a predetermined purity range to make the test result more accurate.

In some embodiments, when the carbon dioxide purity in the supercritical CO₂ corrosion test device measured by the impurity ingredient measuring components 10 is lower than the target value, the controller 80 controls increasing of the flow rate of the carbon dioxide of the supercritical CO₂ corrosion test device by replacing the carbon dioxide in the supercritical CO₂ corrosion test device with a high purity carbon dioxide, so as to increase the carbon dioxide purity in the supercritical CO₂ corrosion test device; and when the carbon dioxide purity in the supercritical CO₂ corrosion test device measured by the impurity ingredient measuring components 10 is higher than the target value, the controller 80 controls decreasing of the flow rate of the carbon dioxide of the supercritical CO₂ corrosion test device by stopping replacing the carbon dioxide in the supercritical CO₂ corrosion test device, so as to decrease the carbon dioxide purity in the supercritical CO₂ corrosion test device.

In the embodiments where the supercritical CO₂ corrosion test device is provided with a booster pump (as mentioned below, a booster pump 2121), the controller 80 may control the start and stop of the booster pump according to the CO₂ purity to achieve the goal of replacing the carbon dioxide in the corrosion test device system so as to adjust the carbon dioxide purity in the corrosion test device.

When the impurity ingredient measuring components 10 detects that the carbon dioxide purity in the supercritical CO₂ corrosion test device is lower than the target value, the controller 80 controls the booster pump of the supercritical CO₂ corrosion test device to start or increase power to increase the flow rate of CO₂ to replace the carbon dioxide in the supercritical CO₂ corrosion test device with a high purity carbon dioxide rapidly, thereby increasing the carbon dioxide purity in the supercritical CO₂ corrosion test device. When the impurity ingredient measuring components 10 detects that the carbon dioxide purity in the supercritical CO₂ corrosion test device is higher than the target value, the controller 80 controls the booster pump to stop or reduce power to decrease the flow rate of CO₂ to stop replacing the carbon dioxide in the supercritical CO₂ corrosion test device, thereby reducing the carbon dioxide purity in the supercritical CO₂ corrosion test device.

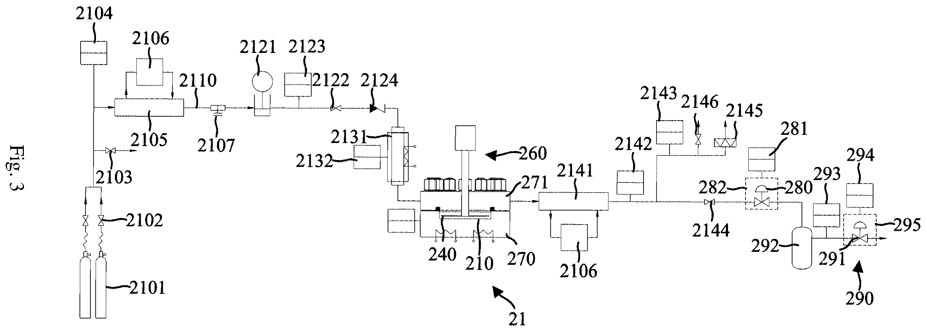

Fig. 3 is a schematic diagram showing the principle of a supercritical CO₂ corrosion test device according to an embodiment of the present application. As shown in Fig. 3, the supercritical CO₂ corrosion test device comprises a sample loading device 21. The sample loading device 21 is configured to have a sample chamber 210 for arranging a sample 250, wherein the sample chamber 210 is also used for receiving a supercritical CO₂ fluid to carry out a corrosion test on the sample 250 in the sample chamber 210 under a supercritical CO₂ atmosphere.

In order to achieve the high pressure condition in the sample chamber 210, the supercritical CO₂ corrosion test device comprises a pressure adjusting valve 280, wherein the pressure adjusting valve 280 is disposed downstream of the sample chamber 210 along the flow direction of the supercritical CO₂ fluid for adjusting the pressure of the sample chamber 210, such that the test on the sample 250 in the sample chamber 210 is carried out under a first predetermined pressure.

The CO₂ gas flowing out of the pressure adjusting valve 280 directly flows into the sample introduction pipeline 91 or into the atmospheric environment. When it is needed to measure the purity, the CO₂ gas flowing out of the pressure adjusting valve 280 directly flows into the sample introduction pipeline 91; and when it is not needed to measure the purity, the CO₂ gas flowing out of the pressure adjusting valve 280 directly flows into the atmospheric environment.

The inventors of the present application have found that if the pressure on one side of the pressure adjusting valve 280 is the same as or slightly higher than that of the atmospheric environment, when the pressure on the other side is maintained up to 25 MPa, due to the sharp drop in CO₂ pressure in the valve, CO₂ will be transitioned from a liquid phase to a gas phase, absorbing a large amount of heat, such that the valve temperature decreases drastically and the valve is likely to freeze, resulting in inaccurate adjustment to the pressure in the sample chamber 210 by the pressure adjusting valve 280.

In related technologies, in order to solve the above problem, an electric heating element is provided to heat the pressure adjusting valve 280, so as to prevent it from freezing. The inventors of the present application have found that the temperature of the pressure adjusting valve 280 decreases very suddenly and the electric heating element has low heating rate and slow response speed, so it is difficult to provide sufficient heat to prevent the temperature of the pressure adjusting valve 280 from decreasing drastically in a short time.

In view of this problem, the supercritical CO₂ corrosion test device further comprises an auxiliary pressure adjusting element 290. The auxiliary pressure adjusting element 290 is used for increasing the pressure at the outlet of the pressure adjusting valve 280. In the embodiments of the present application, the pressure difference between the inlet and outlet of the pressure adjusting valve 280 is reduced by providing the auxiliary pressure adjusting element 290 to increase the pressure at the outlet of the pressure adjusting valve 280. The drastic decrease in temperature of the pressure adjusting valve 280 due to the vaporization phase transition of the supercritical CO₂ at the pressure adjusting valve 280 is avoided, which is advantageous for ensuring the accurate control over the pressure of the sample chamber 210 by the pressure adjusting valve 280.

As shown in Fig. 3, the auxiliary pressure adjusting element 290 comprises an auxiliary pressure adjusting valve 291 (such as a back pressure valve). The auxiliary pressure adjusting valve 291 is disposed downstream of the pressure adjusting valve 280 along the flow direction of the supercritical CO₂ fluid. The supercritical CO₂ fluid from the outlet of the pressure adjusting valve 280 flows to the inlet of the auxiliary pressure adjusting valve 291, and flows from the outlet of the auxiliary pressure adjusting valve 291 to the atmospheric environment. The auxiliary pressure adjusting valve 291 is configured to adjust the pressure at the inlet side of the auxiliary pressure adjusting valve 291 (i.e., the outlet side of the pressure adjusting valve 280) to a second predetermined pressure, wherein the second predetermined pressure is less than the first predetermined pressure and greater than the atmospheric pressure.

By adjusting the pressure at the outlet of the pressure adjusting valve 280 to the second predetermined pressure greater than the atmospheric pressure, the auxiliary pressure adjusting valve 291 reduces the pressure difference between the inlet of the pressure adjusting valve 280 and the outlet of the pressure adjusting valve 280.

In such embodiments, the CO₂ gas flows from the outlet of the auxiliary pressure adjusting valve 291 to the measured sample gas inlet 912 of the sample introduction pipeline 91 or the atmospheric environment. When it is needed to measure the purity, the CO₂ gas flowing out of the auxiliary pressure adjusting valve 291 directly flows into the sample introduction pipeline 91; and when it is not needed to measure the purity, the CO₂ gas flowing out of the auxiliary pressure adjusting valve 291 directly flows into the atmospheric environment.

As shown in Fig. 3, the auxiliary pressure adjusting element 290 further comprises a pressure stabilization vessel 292. The pressure stabilization vessel 292 is disposed in a flow path between the pressure adjusting valve 280 and the auxiliary pressure adjusting valve 291, and the pressure in the pressure stabilization vessel 292 is adjusted by the auxiliary pressure adjusting valve 291. It is more advantageous for the auxiliary pressure adjusting valve 291 to adjust pressure when providing the pressure stabilization vessel 292.

In some embodiments, the pressure stabilization vessel 292 may be a pressure bearing vessel which may bear a pressure of 20 MPa or more.

In some embodiments, as shown in Fig. 3, the auxiliary pressure adjusting element 290 further comprises a pressure measuring element 293. The pressure measuring element 293 is disposed in the flow path between the pressure stabilization vessel 292 and the auxiliary pressure adjusting valve 291, for measuring the pressure at the inlet of the auxiliary pressure adjusting valve 291, so as to adjust the opening degree of the auxiliary pressure adjusting valve 291 according to the pressure measured by the pressure measuring element 293.

In some embodiments, as shown in Fig. 3, the auxiliary pressure adjusting element 290 further comprises an auxiliary temperature measuring element 294 and an auxiliary electric heating element 295. The auxiliary temperature measuring element 294 is used for measuring the temperature of the auxiliary pressure adjusting valve 291. The auxiliary electric heating element 295 heats the auxiliary pressure adjusting valve 291 according to the temperature measured by the auxiliary temperature measuring element 294, so as to heat the auxiliary pressure adjusting valve 291 to a predetermined temperature.

The temperature of the pressure adjusting valve 280 measured by the auxiliary temperature measuring element 294 and the predetermined temperature serve as a basis for heating the auxiliary pressure adjusting valve 291 with the auxiliary electric heating element 295. Such a configuration is helpful for maintaining the temperature of the auxiliary pressure adjusting valve 291 at the predetermined temperature, such that the effect of adjusting the pressure of the auxiliary pressure adjusting valve 291 is better. Even if the vaporization phase transition of the supercritical CO₂ occurs in the auxiliary pressure adjusting valve 291 (if the vaporization phase transition of the supercritical CO₂ would occur), it is also possible to raise the temperature of the auxiliary pressure adjusting valve 291 slowly by providing heat. Because the pressure adjusted by the auxiliary temperature measuring element 294 is the pressure at the inlet side of the auxiliary pressure adjusting valve 291, it is not necessary to be very accurate. Even if the vaporization phase transition of the supercritical CO₂ occurs in the auxiliary pressure adjusting valve 291, such that the auxiliary pressure adjusting valve 291 freezes, it will not influence the effect of the auxiliary temperature measuring element 294 in adjustment to the pressure at the inlet side of the auxiliary pressure adjusting valve 291.

As shown in Fig. 3, the supercritical CO₂ corrosion test device further comprises a first temperature measuring element 281 and a first electric heating element 282. The first temperature measuring element 281 is used for measuring the temperature of the pressure adjusting valve 280. The first electric heating element 282 heats the pressure adjusting valve 280 according to the temperature measured by the first temperature measuring element 281, so as to heat the pressure adjusting valve 280 to a predetermined temperature. In the embodiments of the present application, by providing the auxiliary pressure adjusting element 290 to reduce the pressure difference between the inlet and outlet of the pressure adjusting valve 280, the phase transition therein, which may cause sudden drop in temperature and freezing, is avoided. When the temperature of the pressure adjusting valve 280 is slightly decreased due to the decrease in pressure of the supercritical CO₂ fluid, the use of the first temperature measuring element 281 and the first electric heating element 282 is helpful for maintaining the present application of the pressure adjusting valve 280 at the predetermined temperature, making the effect of the pressure adjusting valve 280 in adjustment to the pressure of the sample chamber 210 better.

In some embodiments, the predetermined temperature may be higher than the ambient temperature, which is advantageous for ensuring the pressure adjusting effects of the pressure adjusting valve 280 and the auxiliary pressure adjusting element 290. For example, the predetermined temperature may be 30 to 50°C.

In some embodiments, the second predetermined pressure may be greater than the corresponding saturated vapor pressure of CO₂ at the ambient temperature. At the ambient temperature of 20°C, the saturated vapor pressure of carbon dioxide is about 5 MPa. When the second predetermined pressure may be greater than the corresponding saturated vapor pressure of CO₂ at the ambient temperature, there is no vigorous vaporization of CO₂ in the pressure adjusting valve 280, and accordingly there is no vigorous heat absorption, and the phenomenon of sudden temperature drop and freezing will not occur.

Therefore, when the first electric heating element 282 heats the pressure adjusting valve 280 to the predetermined temperature, and the auxiliary pressure adjusting valve 291 adjusts the pressure at the outlet of the pressure adjusting valve 280 to be 5 MPa or more, freezing due to overly low temperature of the pressure adjusting valve 280 can be effectively avoided, ensuring the accurate adjustment to the pressure of the sample chamber 210 by the pressure adjusting valve 280.

After test, it is found that in the case where the pressure at the outlet of the pressure adjusting valve 280 has not been increased with the auxiliary pressure adjusting valve 291, the pressure adjusting accuracy of the pressure adjusting valve 280 is ±1 MPa; and when the pressure at the outlet of the pressure adjusting valve 280 is increased with the auxiliary pressure adjusting valve 291, the pressure adjusting accuracy of the pressure adjusting valve 280 can be ±0.5 MPa. Thus, in the present application, the pressure adjusting accuracy of the pressure adjusting valve 280 is increased by providing the auxiliary pressure adjusting valve 291.

In some embodiments, the supercritical CO₂ corrosion test device further comprises a carbon dioxide gas supply 2101, a gas introduction pipeline 2110, a precooling element 2105, a booster pump 2121, a preheating element 2131 and a cooling element 2141.

The gas introduction pipeline 2110 is in communication with a gas introduction opening 220, and the carbon dioxide gas supply 2101 is used for provide gaseous carbon dioxide to the gas introduction pipeline 2110 to input the gaseous carbon dioxide into the gas introduction pipeline 2110. The booster pump 2121, the precooling element 2105, the preheating element 2131 and the cooling element 2141 are disposed in the gas introduction pipeline 2110 respectively. The carbon dioxide gas supply 2101 may be a carbon dioxide gas cylinder. The gas introduction pipeline 2110 is a high pressure pipeline.

The booster pump 2121 is used for pressurizing the liquid carbon dioxide in the gas introduction pipeline 2110 to a test pressure of the supercritical CO₂ corrosion test, so as to provide the high pressure supercritical CO₂ required by the test. Since the booster pump 2121 can only drive liquid carbon dioxide, the precooling element 2105 is provided upstream of the booster pump 2121 for decreasing the temperature of the carbon dioxide in the gas introduction pipeline 2110 to liquefy the gaseous carbon dioxide, such that the carbon dioxide may be conveniently driven by the booster pump 2121.

The preheating element 2131 is provided downstream of the booster pump 2121 for preheating the supercritical CO₂ to a test temperature close to the supercritical CO₂ corrosion test, so as to provide high temperature and high pressure supercritical CO₂, for example at 600°C, required by the test. The preheated high temperature and high pressure supercritical CO₂ enters the sample chamber 210, is further heated in the sample chamber 210 to the test temperature, and flows out of the sample chamber 210 after flowing through the sample 250. The preheating element 2131 may be an electric heating element.

The cooling element 2141 is disposed in a pipeline downstream of the sample chamber 210 for cooling the high temperature supercritical CO₂ to a temperature of 40°C or less, so as to avoid adverse effect on downstream valves. The supercritical CO₂ flowing out of the cooling element 2141 flows in the pressure adjusting valve 280, the pressure stabilization vessel 292 and the auxiliary pressure adjusting element 290 sequentially, and then enters the atmospheric environment.

In some embodiments, the gas introduction pipeline 2110 is also provided with a gas source valve 2102, and the supply or discontinuation of gaseous carbon dioxide is achieved by opening or closing the gas source valve 2102.

In some embodiments, the supercritical CO₂ corrosion test device further comprises a displacement valve 2103, wherein the displacement valve 2103 is disposed in a pipeline in parallel with the gas introduction pipeline 2110 for displacing the air impurity introduced in the process of changing the carbon dioxide gas supply 2101.

In some embodiments, the gas introduction pipeline 2110 is further provided with a first pressure measuring element 2104, wherein the first pressure measuring element 2104 is disposed upstream of the precooling element 2105 for determining the pressure at the inlet of the precooling element 2105 and alerting when the pressure at the inlet of the precooling element 2105 is too low. The first pressure measuring element 2104 may be a digital pressure sensor.

In some embodiments, the supercritical CO₂ corrosion test device further comprises a cool water supply element 2106, wherein the cool water supply element 2106 is disposed on the precooling element 2105 for providing a cooling capacity required by the precooling element 2105, so as to control the temperature of the carbon dioxide in the gas introduction pipeline 2110.

In some embodiments, the gas introduction pipeline 2110 is further provided with a filter element 2107, wherein the filter element 2107 is disposed between the precooling element 2105 and the booster pump 2121 for removing the impurity in the liquid carbon dioxide, so as to obtain a clean liquid carbon dioxide. The filter element 2107 may be a 15 µm sintered T-type filter.

In some embodiments, the gas introduction pipeline 2110 is further provided with a second pressure measuring element 2123, wherein the second pressure measuring element 2123 is disposed downstream of the booster pump 2121 for determining the pressure at the outlet of the booster pump 2121 and alerting when the pressure at the outlet of the booster pump 2121 is too high, so as to prevent failure of the booster pump 2121 due to the overly high pressure at the outlet.

In some embodiments, the gas introduction pipeline 2110 is further provided with a valve 2122 and a check valve 2124, wherein the valve 2122 and the check valve 2124 are disposed downstream of second pressure measuring element 2123 sequentially. Here, the valve 2122 is used for controlling the flow of the liquid carbon dioxide in the gas introduction pipeline 2110; and the check valve 2124 is used for preventing the liquid carbon dioxide from flowing in the gas introduction pipeline 2110 in the reverse direction. The second pressure measuring element 2123 may be a digital pressure sensor.

In some embodiments, the supercritical CO₂ corrosion test device further comprises a temperature control element 2132, wherein the temperature control element 2132 is disposed on the preheating element 2131 for displaying the temperature of the preheating element 2131, such that the preheating element 2131 adjusts the heating power according to the temperature measured by the temperature control element 2132.

In some embodiments, the cooling element 2141 is provided with a cool water supply element 2106, wherein the cool water supply element 2106 provides a cooling capacity source to the cooling element 2141.

In some embodiments, the gas introduction pipeline 2110 is further provided with a second temperature measuring element 2142, wherein the second temperature measuring element 2142 is disposed downstream of the cooling element 2141. The second temperature measuring element 2142 is used for determining the temperature of the cooled supercritical CO₂ and alerting when the temperature of the cooled supercritical CO₂ exceeds 40°C.

In some embodiments, the supercritical CO₂ corrosion test device further comprises a third pressure measuring element 2143, an atmospheric relief valve 2146 and a rupture disc 2145. The third pressure measuring element 2143, the atmospheric relief valve 2146 and the rupture disc 2145 are disposed sequentially in another pipeline in parallel with the gas introduction pipeline 2110. Here, the third pressure measuring element 2143 is used for measuring the pressure in this parallel pipeline. The atmospheric relief valve 2146 is controlled to open or close according to the pressure measured by the third pressure measuring element 2143. The discharging of CO₂ from the pipeline is controlled by opening or closing the atmospheric relief valve 2146, so as to achieve the adjustment to the pressure in this parallel pipeline. The third pressure measuring element 2143 may be a digital pressure sensor. The rupture disc 2145 ruptures at predetermined temperature and pressure to relief pressure, so as to prevent the device failure due to overly high pressure in this parallel pipeline.

In some embodiments, the gas introduction pipeline 2110 is further provided with a vent valve 2144, wherein the vent valve 2144 is disposed downstream of the second temperature measuring element 2142, and the flow of the supercritical CO₂ is controlled by controlling the vent valve 2144 to open or close.

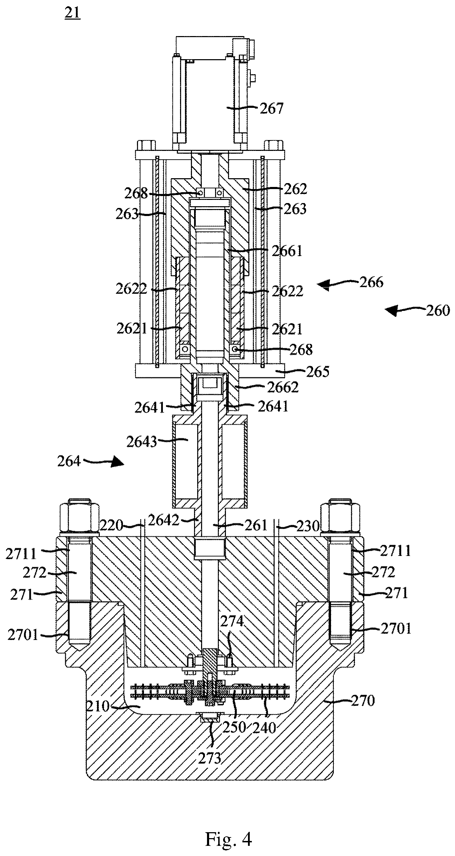

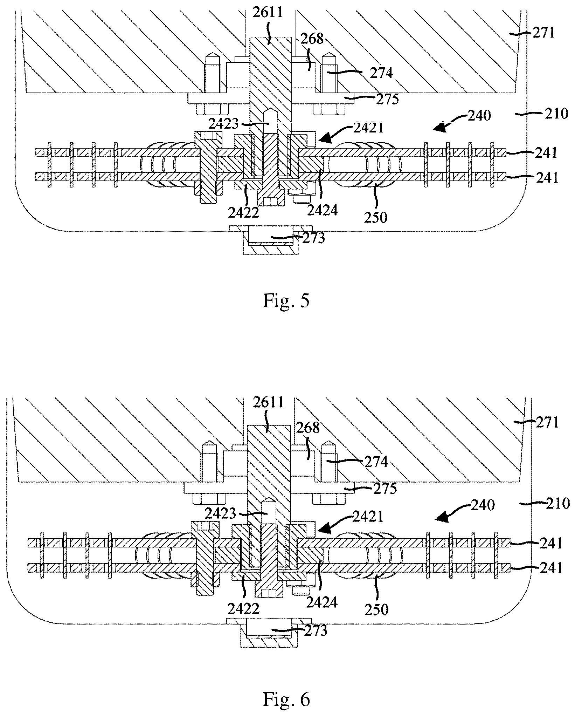

Fig. 4 is a schematic sectional view of a sample loading device according to an embodiment of the present application, and Fig. 5 is a schematic partially enlarged view of the sample chamber of the sample loading device in Fig. 4. As shown in Fig. 4 and Fig. 5, the sample loading device 21 may comprise: a sample chamber 210, a gas introduction opening 220, a gas discharge opening 230, a sample holding component 240, and a rotation driving element 260. The gas introduction opening 220 is used for introducing a supercritical CO₂ gas into the sample chamber 210. The gas discharge opening 230 is used for flowing the supercritical CO₂ gas in the sample chamber 210 out of the sample chamber 210. The sample holding component 240 is disposed in the sample chamber 210 for holding a plurality of samples 250. The rotation driving element 260 is used for driving the sample holding component 240 to rotate, such that the supercritical CO₂ in the sample chamber 210 can flow at a high speed along the surface of the sample 250.

In the embodiments of the present application, by driving the sample holding component 240 to rotate with the rotation driving element 260, the sample 250 has a certain linear velocity, so as to increase the relative flow rate of the supercritical CO₂ in the sample chamber 210 with respect to the sample 250, and allow the supercritical CO₂ to flow at a high speed along the surface of the sample 250, such that the high flow rate supercritical CO₂ corrosion test on the sample 250 can be achieved.

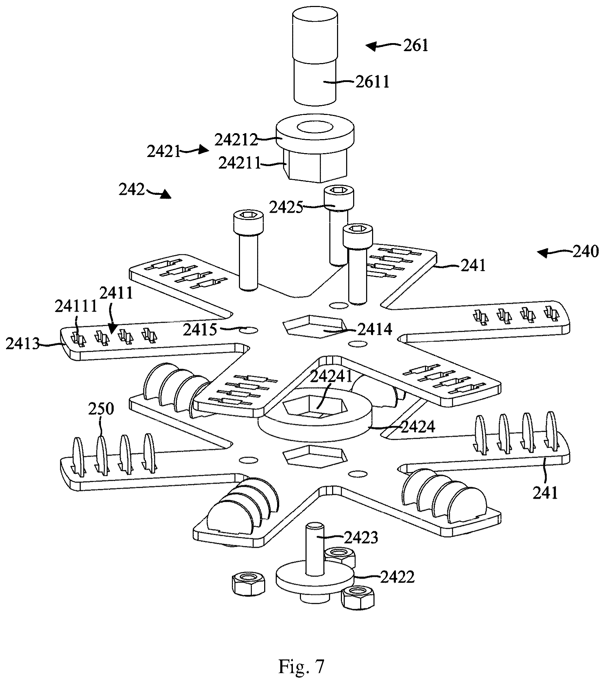

Fig. 6 is a schematic sectional view of a sample holding component according to an embodiment of the present application, and Fig. 7 is a schematic exploded view of the sample holding component according to an embodiment of the present application. As shown in Fig. 6 and Fig. 7, in some embodiments, the sample holding component 240 may comprise two holding elements 241 disposed to face each other and a connecting assembly 242. A plurality of positioning grooves 24111 are formed on each of the holding elements 241. The samples 250 are disc-shaped samples 250. Each disc-shaped sample 250 is inserted and kept in corresponding positioning grooves 24111 of the two holding elements 241. The connecting assembly 242 is used for connecting the two holding elements 241 to the rotation driving element 260. In such embodiments, the rotation driving element 260 is connected to the two holding elements 241 through the connecting assembly 242, thereby achieving the rotation of the disc-shaped sample 250 inserted and kept in the corresponding positioning grooves 24111 of the two holding elements 241, such that the disc-shaped sample 250 has a certain linear velocity. The rotation driving element 260 drives the disc-shaped sample 250 to move relative to the supercritical CO₂, so it is not necessary to directly provide a high flow rate CO₂, thereby reducing the load on devices for the filling, storing and heat exchanging of CO₂, and greatly reducing the manufacture cost of the devices.

In some embodiments, the two holding elements 241 have the same structure. The holding elements 241 may be an upper six-claw clamp plate and a lower six-claw clamp plate.

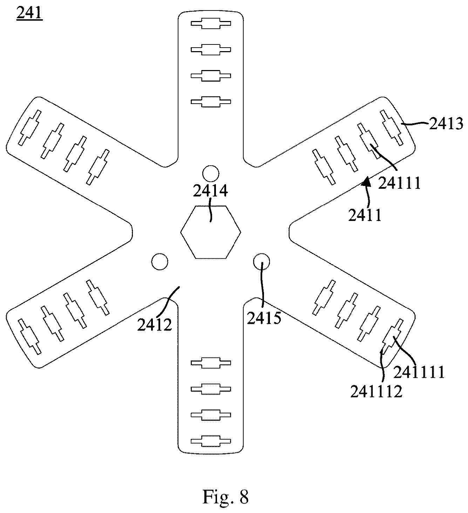

Fig. 8 is a schematic top view of a holding element according to an embodiment of the present application. As shown in Fig. 8, a plurality of positioning groove groups 2411 are formed on the holding element 241, and are distributed along its circumference, wherein each positioning groove group 2411 comprises a plurality of positioning grooves 24111 at different distances from the rotation axis of the holding element 241; and the distance between one positioning groove 24111 in each positioning groove group 2411 and the rotation axis is the same as the distance between a corresponding one positioning groove 24111 in any other positioning groove group 2411 and the rotation axis.

Because the distance between one positioning groove 24111 in each positioning groove group 2411 and the rotation axis is the same as the distance between a corresponding one positioning groove 24111 in any other positioning groove group 2411 and the rotation axis, the plurality of positioning groove groups 2411 allow a plurality of samples 250 to have the same linear velocity, thereby increasing the number of samples 250 having the same linear velocity.

Because each positioning groove group 2411 comprises a plurality of positioning grooves 24111 at different distances from the rotation axis of the holding element 241, and a plurality of positioning grooves 24111 in each positioning groove group 2411 are disposed on the same horizontal axis passing through the rotation axis, a plurality of samples 250 positioned in one positioning groove group 2411 have the same angular velocity and different linear velocities, such that corrosion tests on the samples under different supercritical CO₂ flow rate conditions may be simulated in one test.

In some embodiments, each positioning groove 24111 comprises a center groove area 241111 and end groove areas 241112 disposed at two ends of the center groove area 241111 along its length direction. The width of the center groove area 241111 is greater than that of the end groove areas 241112. The length direction of the positioning groove 24111 is perpendicular to the radial direction of the holding element 241. The entire length of the positioning groove 24111 is less than the diameter of the disc-shaped sample 250, and the width of the end groove areas 241112 is greater than the thickness of the disc-shaped sample 250, such that when the disc-shaped sample 250 is inserted in the positioning groove 24111, the contact surface between the positioning groove 24111 and the disc-shaped sample 250 is a "line contact" rather than a "surface contact" , thereby reducing the contact area between the positioning groove 24111 and the disc-shaped sample 250, such that the sample positioning groove 24111 minimally influences the disc-shaped sample 250 during the test; and the contact area between the disc-shaped sample 250 and the supercritical CO₂ is larger to allow the disc-shaped sample 250 and the supercritical CO₂ to react more sufficiently.

As shown in Fig. 6, the holding element 241 comprises a core portion 2412 and a plurality of extending portions 2413 along the periphery of the core portion 2412 and connected to the core portion 2412, wherein each positioning groove group 2411 is disposed in one corresponding extending portion 2413. Such a configuration is advantageous for the holding element 241 to reduce the overall weight of the holding element 241 while ensuring that the number of the samples 250 remains unchanged, thereby reducing unnecessary friction and inertia when the rotation driving element 260 drives the holding element 241 to rotate, and further reducing the energy consumption required when the rotation driving element 260 drives the holding element 241 to rotate. Meanwhile, the reduction of the overall weight of the holding element 241 also contributes to making the rotation driving element 260 more stable when driving the holding element 241, thereby ensuring the stability when the rotation driving element 260 drives the sample 250 to rotate.

In some embodiments, the extending portions 2413 extend radially outward from the core portion 2412.

In some embodiments, each of the extending portions 2413 has the same shape, and the extending portions 2413 are disposed axis symmetrically with respect to the rotation axis.

In some embodiments, the number of the extending portions 2413 may be 6, and the angle between two adjacent extending portions 2413 is 60°. Such a configuration allows the holding element 241 to bear balanced force along the horizontal direction, and prevents the holding element 241 from tilting in the horizontal direction, thereby making the holding element 241 achieve stable horizontal rotation.

In some embodiments, the number of the positioning grooves 24111 in each extending portion 2413 may be 2 to 6.

In some embodiments, the distance between the positioning groove 24111 in each extending portion 2413 furthest away from the rotation axis and the rotation axis may be 80 mm, and the distance between two adjacent positioning grooves 24111 in the same extending portion 2413 may be one tenth of the above distance, i.e., 8 mm.

In some embodiments, the rotational speed at which the rotation driving element 260 drives the sample holding component 240 to rotate may reach 1000 r/min, and may be, for example, 1800 r/min. The flow rate of the sample 250 held by the sample holding component 240 relative to the supercritical CO₂ may be greater than 5 m/s.

As shown in Fig. 7, the rotation driving element 260 comprises a rotation shaft 261, and a center mounting hole 2414 is formed in each holding element 241. The connecting assembly 242 may comprise: a connecting nesting element 2421, a connection fitting element 2422 and a fastening element 2423. The connecting nesting element 2421 and the connection fitting element 2422 are disposed on two opposite sides of the two holding elements 241 respectively, the connecting nesting element 2421 passes into two center mounting holes 2414, and the connecting nesting element 2421 is detachably connected to the rotation shaft 261. The fastening element 2423 detachably connects the connection fitting element 2422 to the rotation shaft 261, so as to connect the two holding elements 241 while connecting the two holding elements 241 to the rotation shaft 261. The connecting assembly 242 connects the two holding elements 241, thereby achieving the fixation of the two holding elements 241 and the stable clamping of the sample 250. Meanwhile, the connecting assembly 242 connects the two holding elements 241 to the rotation driving element 260, thereby achieving the driving of the two holding elements 241 by the rotation driving element 260. As such, while the sample holding component 240 and the rotation shaft 261 are assembled and disassembled, the sample 250 and the sample holding component 240 are assembled and disassembled, thereby simplifying the assembly and disassembly operations.

In some embodiments, the rotation shaft 261 is provided with a rotation shaft connecting port 2611, wherein the rotation shaft connecting port 2611 is configured to be a hollow cylinder for achieving the detachable connection with the connecting nesting element 2421 and the fastening element 2423. In some embodiments, the rotation shaft connecting port 2611 is inserted inside the connecting nesting element 2421, and the rotation shaft connecting port 2611 is detachably connected to the connecting nesting element 2421. In some embodiments, the detachable connection between the rotation shaft connecting port 2611 and the connecting nesting element 2421 may be a threaded connection. In some embodiments, the detachable connection between the rotation shaft connecting port 2611 and the connecting nesting element 2421 may be a threaded connection.

In some embodiments, the connecting nesting element 2421 comprises a nesting element body 24211 and a clamping portion 24212 disposed on the nesting element body. The outer diameter of the clamping portion 24212 is greater than the inner diameter of the center mounting hole 2414 for clamping connection at the side of the holding element 241 close to the rotation shaft 261. The connection fitting element 2422 is a circular ring having an outer diameter greater than the inner diameter of the center mounting hole 2414. The clamping portion 24212 and the connection fitting element 2422 are used for closely clamping the two holding elements 241 respectively. The nesting element body 24211 has a size less than the inner diameter of the center mounting hole 2414 such that it can pass into the center mounting hole 2414. The rotation shaft connecting port 2611 of the rotation shaft 261 passes into the nesting element body 24211, and is threaded connected to the nesting element body 24211; and the fastening element 2423 passes through the connection fitting element 2422 into the rotation shaft connecting port 2611 from the other side, and is threaded connected to the rotation shaft connecting port 2611.

In some embodiments, the fastening element 2423 may be a lock screw, and the connection fitting element 2422 may be a lock gasket. For example, the fastening element 2423 is an M6 hexagon socket head lock screw.

The holding element 241 may be a ceramic element.

As shown in Fig. 7, the connecting assembly 242 further comprises a gasket 2424 disposed between the two holding elements 241, and the gasket 2424 is provided with a gasket mounting hole 24241. The connecting nesting element 2421 passes into the two center mounting holes 2414 and the gasket mounting hole 24241. A plurality of connection elements 2425 connect the two holding elements 241 at the radially outside of the gasket 2424. The gasket 2424 is provided between the two holding elements 241, such that the distance between the two holding elements 241 is maintained constant when driving the sample holding component 240 to rotate, ensuring the stability of the sample 250 between the two holding elements 241. Meanwhile, by configuring the height of the gasket 2424, the sample 250 may be movably disposed in the positioning grooves 24111 of the two holding elements 241, so as to prevent the sample 250 from bearing a large stress applied by the holding elements 241, thereby avoiding the damage of the sample 250.

In some embodiments, the cross sections of the center mounting hole 2414, the gasket mounting hole 24241 and the nesting element body 24211 are all hexagonal to prevent the nesting element body 24211 and the holding elements 241 from rotating relative to each other.

In some embodiments, the holding elements 241 are provided with a plurality of holding element outside mounting holes 2415 at the radially outside of the gasket 2424, and each mounting hole of the holding element 241 is used for one connection element 2425 to pass into.

In some embodiments, the connection elements 2425 may be screws and nuts. For example, when the screws are M6 hexagon socket head fixing screws, the nuts are M6 hexagon socket head fixing nuts.

As shown in Fig. 4, the rotation driving element 260 may further comprise: a drive element 267, a rotation cylinder 262 and a magnet 2621. The drive element 267 drives the rotation cylinder 262 to rotate, and the magnet 2621 is disposed on the rotation cylinder 262. The rotation shaft 261 is disposed on the radially inside of the rotation cylinder 262 and the magnet 2621 to rotate along with the rotation cylinder 262 under the magnetic force of the magnet 2621. A bearing 268 is provided between the rotation shaft 261 and the rotation cylinder 262. The drive element 267 may be a rotating motor, and theoretically, any linear velocity of the sample relative to the supercritical CO₂ may be obtained by adjusting the rotational speed of the rotating motor.

In some embodiments, the rotation driving element 260 may further comprise a connection cylinder 2622 connected to the rotation cylinder 262, and the magnet 2621 may be disposed on the connection cylinder 2622.

In some embodiments, the rotational speed at which the rotation driving element 260 drives the rotation cylinder 262 to rotate may be more than 1000 r/min (for example, 1800 r/min), and the continuous rotation time may reach several thousand hours (for example, one year). When the rotation cylinder 262 rotates at a high speed for an extended time period, the temperature of the magnet 2621 disposed on the rotation cylinder 262 will gradually increase, and demagnetization will occur when the temperature of the magnet 2621 is too high, such that the drive element 267 cannot drive the rotation shaft 261 to rotate. In view of this problem, the rotation driving element 260 may further comprise a rotation cylinder cooling element 263, wherein the rotation cylinder cooling element 263 is used for cooling the rotation cylinder 262 and the magnet 2621 to avoid the demagnetization of the magnet 2621, thereby ensuring that the sample loading device 21 can be used for an extended time period.

As shown in Fig. 4, the sample loading device 21 may further comprise a body 270 and a cover 271. The body 270 has a top opening. The cover 271 is used for enclosing the top opening so as to form the sample chamber 210 together with the body 270. The rotation shaft 261 passes through the cover 271 into the sample chamber 210. A bearing 268 is provided between the rotation shaft 261 and the cover 271. The bearing 268 may be disposed on a bearing bracket 275, and connected to the cover 271 by a fastening element 274.

The sample 250 may be a 16 mm disc sample with a thickness of 1 mm.

In the sample loading device 21 of the embodiments of the present application, the volume of the sample chamber 210 can be configured to be small, such as about 1 L, which may effectively reduce heat loss, reduce carbon dioxide consumption, and eliminate interference caused by difference in test temperature of the samples at different locations due to overly large volume and overly long test segment length.

When the volume of the sample chamber 210 is configured to be about 1 L, the theoretical value of the carbon dioxide consumption in each operation of the supercritical CO₂ corrosion test is 0.1 kg; as compared to the high flow rate supercritical carbon dioxide corrosion test device for providing high flow rate CO₂ in related technologies, the theoretical value of the carbon dioxide consumption in each operation of the former is one thousandth of that of the latter.

The rotation driving element 260 further comprises a rotation shaft cooling element 264 for cooling the rotation shaft 261. The rotation shaft cooling element 264 is connected to the cover 271 below the rotation cylinder 262. The rotation shaft cooling element 264 is used for decreasing the surface temperature of the rotation shaft 261 to prevent the heat in the sample chamber 210 from diffusing to the magnet 2621, thereby avoiding the demagnetization of the magnet 2621.

The gas introduction opening 220 and the gas discharge opening 230 are formed on the cover 271 and penetrate the cover 271 respectively. The gas introduction opening 220 and the gas discharge opening 230 may be disposed at two sides of the rotation shaft 261 in the radial direction.

As shown in Fig. 4, in some embodiments, the device further comprises a fixing element 272. The cover 271 is provided with a through hole 2711, and the body 270 is provided with a groove 2701 corresponding to the through hole 2711 of the cover 271. The fixing element 272 passes through the through hole 2711 of the cover 271 and is detachably connected to the corresponding groove 2701 of the body 270 for achieving the assembly and disassembly of the cover 271 and the body 270.

In some embodiments, the body 270 is provided with a heating element and a temperature measuring element, wherein the heating element is used for heating the sample chamber 210, such that the supercritical CO₂ in the sample chamber 210 is at a predetermined test temperature; and the temperature measuring element is used for measuring the temperature of the sample chamber 210, such that the heating element adjusts the heating power according to the temperature measured by the temperature measuring element. The heating element of the body 270 may be an electric heating element.

In some embodiments, the fixing element 272 may be a screw and a nut for achieving firm connection between the cover 271 and the body 270.

As shown in Fig. 5, in some embodiments, the sample chamber 210 is provided with a static sample groove 273 on its bottom wall, wherein the static sample groove 273 is disposed directly below the rotation shaft 261. The static sample groove 273 is used for placing the sample 250. During the test, because the sample 250 is positioned directly below the rotation shaft 261 where the gas flow is minimal in the sample chamber 210, the sample 250 can be considered as being in a static state.

As shown in Fig. 4, the rotation driving element 260 further comprises a drive mounting element 265 and a sleeve 266. The drive element 267 and the rotation cylinder cooling element 263 are disposed on the drive mounting element 265. The sleeve 266 is connected to the rotation shaft cooling element 264 and the drive mounting element 265, and the sleeve 266 is disposed between the rotation shaft 261 and the rotation cylinder 262. A bearing 268 is provided between the connection cylinder 2622 and the sleeve 266 to improve the rotation stability of the rotation cylinder 262 and the magnet 2621.

The rotation shaft cooling element 264 is connected to the sleeve 266, and the sleeve 266 and the drive mounting element 265 are supported by the rotation shaft cooling element 264.

The rotation shaft cooling element 264 and the rotation cylinder cooling element 263 may be cooled by water cooling.

In some embodiments, the sleeve 266 forms a first connection portion 2661 and a second connection portion 2662. Here, the first connection portion 2661 is disposed between the rotation shaft 261 and the rotation cylinder 262 as well as the magnet 2621, and the second connection portion 2662 is used for connecting the rotation shaft cooling element 264 to the drive mounting element 265 to achieve stable connection between the rotation shaft cooling element 264 and the drive mounting element 265.

In some embodiments, the rotation shaft cooling element 264 forms a first cooling connection portion 2641, a cooling body 2643 and a second cooling connection portion 2642. The first cooling connection portion 2641 is connected to the second connection portion 2662 of the sleeve 266. The cooling body 2643 is disposed outside the rotation shaft 261 for decreasing the surface temperature of the rotation shaft 261. The second cooling connection portion 2642 is connected to the cover 271.

By providing the first cooling connection portion 2641, the rotation driving element 260 can be mounted on the cover 271 as a whole and the cover 271 and the rotation driving element 260 can be moved as a whole, facilitating the disassembly and assembly of the sample holding component 240; and the heat in the sample chamber 210 will not be transferred to the magnet 2621.

The embodiments of the present application also provides a supercritical CO₂ corrosion test method which uses the supercritical CO₂ corrosion test device of the embodiments of the present application. The test method comprises: introducing a supercritical CO₂ into the sample chamber 210, wherein the supercritical CO₂ flows through the sample chamber 210, the pressure adjusting valve 280 and the auxiliary pressure adjusting element 290 sequentially, and then is discharged to the atmospheric environment; adjusting the pressure adjusting valve 280 such that the pressure in the sample chamber is a first predetermined pressure; adjusting the auxiliary pressure adjusting element 290 such that the pressure at the outlet of the pressure adjusting valve 280 is a second predetermined pressure, wherein the second predetermined pressure is less than the first predetermined pressure and greater than the atmospheric pressure; and then carrying out the supercritical CO₂ corrosion test.

In the method of the embodiments of the present application, the pressure adjusting valve 280 is used to adjust the pressure in the sample chamber 210, such that the pressure in the sample chamber 210 is maintained at the predetermined pressure, and the sample chamber 210 is maintained under a high pressure condition required by the supercritical CO₂ corrosion test; the auxiliary pressure adjusting element 290 is used to increase the pressure at the outlet of the pressure adjusting valve 280 to the second predetermined pressure, so as to reduce the pressure difference between the inlet and outlet of the pressure adjusting valve 280, thereby avoiding the drastic decrease in temperature of the pressure adjusting valve 280 due to the vaporization phase transition of the supercritical CO₂ at the pressure adjusting valve 280, which is advantageous for ensuring the accurate control over the pressure in the sample chamber 210 by the pressure adjusting valve 280 to keep the pressure of the supercritical CO₂ in the sample chamber 210 stable.

In some embodiments, before carrying out the supercritical CO₂ corrosion test, all heating elements are turned on to heat the supercritical CO₂ to a predetermined temperature; the rotation driving element 260 is turned on to drive the sample holding component 240 to rotate at a predetermined rotational speed, and then the supercritical CO₂ corrosion test is started.

The flow process for performing the supercritical CO₂ corrosion test with the measuring system 100 of the present disclosure and the test method will be further described below with reference to the drawings and particular embodiments.
1. Safety check: After the device is powered on, it is checked whether the display status of various measuring elements is normal and whether the water level of the cool water supply element 2106 is normal, whether all the valves are closed, and whether all test parameters and alerting parameters of the device are set normally.
2. Placement of the sample: The sample 250, the sample holding component 240 and the rotation shaft connecting port 2611 on the cover 271 are assembled, and then the sample holding component 240 is placed into the body 270.
3. Sealing of the sample chamber 210: It is checked whether there are solid particles or foreign matter on the contact surface between the cover 271 and the body 270, whether the fixing element 272 is correctly mounted, and the cover 271 is fastened to the body 270 with the fixing element 272 to seal the sample chamber 210.
4. Introduction of carbon dioxide: The carbon dioxide gas supply 2101 provides a gaseous carbon dioxide to the gas introduction pipeline 2110, the gas source valve 2102 is opened, introduced air is discharged through the displacement valve 2103, and it is observed whether the display of the first pressure measuring element 2104 is normal. The valve 2122 is slowly opened to inject carbon dioxide into the sample chamber 210, and it is observed whether the display of the second pressure measuring element 2123 and the third pressure measuring element 2143 is normal, the vent valve 2144 is opened, and the atmospheric relief valve 2146 is opened to purge and replace air in the sample chamber 210.
5. Control of the test condition: The atmospheric relief valve 2146 is closed, the cool water supply element 2106 is powered on, a cooling capacity is provided to the precooling element 2105 and the cooling element 2141, the precooling element 2105 liquefies the gaseous carbon dioxide to a liquid carbon dioxide, and the cooling element 2141 decreases the temperature of the carbon dioxide in the pipeline. All electric heating elements are powered on, and the temperature control elements automatically adjust the power of the electric heating elements according to the predetermined temperature respectively. The booster pump 2121 is turned on, and the rotatable handles of the pressure adjusting valve 280 and the auxiliary pressure adjusting valve 291 are tuned, until the pressure in the sample chamber 210 reaches the predetermined test pressure and the pressure in the pressure stabilization vessel 292 reaches the predetermined pressures respectively. The CO₂ gas flows from the outlet of the auxiliary pressure adjusting valve 291 to the measured sample gas inlet 912 of the sample introduction pipeline 91. The type of the CO₂ sample gas is input into the controller 80, the controller 80 matches the input type of the CO₂ sample gas against the impurity ingredients and the content ranges of the impurity ingredients corresponding to the various types preset therein, so as to control each switching element to switch the fluid communication with the sample introduction pipeline 91 to the impurity ingredient measuring element having a corresponding measuring range in each group of impurity ingredient measuring components 10.
6. Adjustment of the rotational speed: The drive element 267 is turned on, the rotational speed of the drive element 267 is adjusted according to different requirements on the linear velocity, and the test is started.
7. After the test is ended, the drive element 267 is turned off, the gas source valve 2102 is closed, the booster pump 2121 is turned off, and the rotatable handle of the pressure adjusting valve 280 and the rotatable handle of the auxiliary pressure adjusting valve 291 are slowly rotated counterclockwise to allow the CO₂ in the system to be discharged to the atmospheric environment through the pressure adjusting valve 280 and the auxiliary pressure adjusting valve 291 sequentially. When all the pressures measured by the first pressure measuring element 2104, the second pressure measuring element 2123 and the third pressure measuring element 2143 are decreased to 0, the cool water supply element 2106 and various electric heating elements are turned off. After the temperature of the sample chamber 210 is decreased to the ambient temperature, the fixing element 272 is screwed and the cover 271 is opened to take out the sample 250.

It should also be noted that, for the embodiments of the present application, unless contradiction, the embodiments of the present application and the features in the embodiments can be combined with each other to obtain new embodiments.

The above descriptions are only particular embodiments of the present application, and the protection scope of the present application are not limited thereto. The protection scope of the present application should be defined by the following claims.

## Claims

1. A system for measuring a CO₂ purity, wherein the system comprises:
a sample introduction pipeline for receiving a CO₂ sample gas to be measured; and
at least one group of impurity ingredient measuring components, each group of impurity ingredient measuring components comprising a switching element and two impurity ingredient measuring elements for measuring a content of one and the same kind of impurity in the CO₂ sample gas in the sample introduction pipeline,
wherein the two impurity ingredient measuring elements in the same group have different measuring ranges, the switching element is used to select one of the two impurity ingredient measuring elements in the same group to be in fluid communication with the sample introduction pipeline, the switching element is configured to determine whether to switch the fluid communication with the sample introduction pipeline to the other impurity ingredient measuring element in the same group according to the measuring range of the impurity ingredient measuring element currently being in fluid communication with the sample introduction pipeline and a corresponding impurity content in the CO₂ sample gas measured thereby;
wherein the switching element is a three-way valve comprising one inlet end and two outlet ends, wherein the inlet end is in fluid communication with the sample introduction pipeline, and the two outlet ends are in fluid communication with the two impurity ingredient measuring elements in the same group respectively;
the system further comprises:
a pressure measuring element for measuring a pressure value of the CO₂ sample gas entering the sample introduction pipeline; and
a pressure adjusting element for adjusting a pressure of the CO₂ sample gas entering the sample introduction pipeline according to the pressure value measured by the pressure measuring element such that the pressure of the CO₂ sample gas is within a predetermined range;
the system further comprises:
a controller for controlling the switching element in each group of impurity ingredient measuring components to switch the fluid communication with the sample introduction pipeline to an impurity ingredient measuring element having a corresponding measuring range according to a type of the CO₂ sample gas to be measured;
wherein the controller is preset with types of the CO₂ sample gas as well as impurity ingredients and content ranges of the impurity ingredients corresponding to various types; the controller may further comprise an input module for inputting a type of the CO₂ sample gas, and the controller matches the input type of the CO₂ sample gas against the impurity ingredients and the content ranges of the impurity ingredients corresponding to the various types preset therein, so as to control the switching element to switch fluid communication with the sample introduction pipeline to the impurity ingredient measuring element having a corresponding measuring range in each group of impurity ingredient measuring components.

2. The system according to claim 1, wherein the pressure adjusting element comprises: a pressure reducing valve disposed in the sample introduction pipeline and a metal adjusting valve disposed downstream of the pressure reducing valve.

3. The system according to claim 1, wherein the system comprises a plurality of groups of impurity ingredient measuring components for measuring different kinds of impurity ingredients in the CO₂ sample gas respectively, wherein the impurity ingredients include at least one of CO, O₂ and CₙHₘ; and
the plurality of groups of impurity ingredient measuring components are disposed in parallel with each other.

4. The system according to claim 1, wherein the system further comprises: a water content measuring element for measuring a water content in the CO₂ sample gas.

5. The system according to claim 1, wherein the system further comprises:
a standard sample gas introduction pipeline for providing a standard sample having a known impurity content to the at least one group of impurity ingredient measuring components so as to calibrate the at least one group of impurity ingredient measuring components.

6. The system according to claim 1, wherein the system further comprises:
a purge pipeline for providing a purge gas to the sample introduction pipeline and the at least one group of impurity ingredient measuring components.

7. The system according to claim 1, wherein the system further comprises:
a drying element for drying the CO₂ sample gas in the sample introduction pipeline such that the dried CO₂ sample gas enters the at least one group of impurity ingredient measuring components; and/or
a filter element for filtering the CO₂ sample gas in the sample introduction pipeline such that the filtered CO₂ sample gas enters the at least one group of impurity ingredient measuring components.

8. The system according to claim 1, wherein the system further comprises:
a discharge pipeline for discharging a gas from the at least one group of impurity ingredient measuring components to an atmospheric environment.
